# EUROPEAN PATENT SPECIFICATION

(11) **EP 2 978 453 B1**
(45) Date of publication and mention of the grant of the patent: **31.10.2018**
(21) Application number: 14773271.3
(22) Date of filing: 31.03.2014
(51) Int. Cl.: G02C 7/04

(54) **DRUG DELIVERY FROM CONTACT LENSES WITH A FLUIDIC MODULE**
ARZNEIMITTELABGABE AUS KONTAKTLINSEN MIT EINEM FLUIDISCHEN MODUL
ADMINISTRATION DE MÉDICAMENT À PARTIR DE LENTILLES DE CONTACT AVEC UN MODULE FLUIDIQUE

(30) Priority: 29.03.2013 US 201361806538 P
(43) Date of publication of application: 03.02.2016
(73) Proprietor: Onefocus Technology, LLC, Fernandina Beach, FL 32034 (US)
(72) Inventor: WAITE, Steve, Fernandiana Beach, FL 32034 (US); GUPTA, Amitava, Roanoke, VA 24018 (US); SCHNELL, Urban, CH-3053 Munchenbuschesee (CH)
(74) Representative: Clark, Jane Anne
(86) International application number: PCT/US2014/032401
(87) International publication number: WO 2014/161002

(56) References cited:
- EP-A2- 2 639 626
- WO-A2-2010/068281
- WO-A2-2014/117173
- US-A- 5 270 051
- US-A1- 2007 034 533
- US-A1- 2011 244 010
- US-A1- 2011 244 010

## Description

### CROSS-REFERENCE

### BACKGROUND OF THE INVENTION

This invention relates to soft contact lenses.

Prior methods and apparatus of delivery of therapeutic agents can be less than ideal in at least some instances. The release mechanisms of the prior devices and be less than ideal, and provide less than ideal drug release profiles. Although contact lenses have been proposed to release therapeutic agents, the prior contact lenses can provide less than ideal amounts of drug and release profiles.

Administration of drugs to treat eye diseases can be challenging, since normal modes of drug administration fail to deliver therapeutically beneficial doses beyond a short time.

Drugs delivered via eye drops, for example, can be rapidly washed away by the tear film, and only about 1/104 or less of the total amount of the drug delivered via an eye drop reaches the anterior or the posterior chamber.

The total bioavailability of a lipophilic drug can be about 10% in the anterior chamber relative to its concentration delivered as eye drops, while that of hydrophilic drugs and large molecules can be less than 5%.

The total amount that enters the anterior chamber can be further reduced by an order of magnitude since typical eye drops deliver about 60-70 µl, while the eye rapidly removes all fluids in excess of 7 µl.

There still exists an unmet need of an agent to deliver drugs into the anterior and posterior chambers of the eye in a sustained manner.

In light of the above, it would be desirable to have improved release of therapeutic agents from contact lenses. Ideally, such devices will provide improved release of therapeutic agents from contact lenses.

EP 2,639,626 (which published on 18 September 2013 and so can be considered only for the purposes of novelty under Article 54(3) EPC) describes a contact lens incorporating one or more dynamic fluid zones fabricated from a material that is readily deformable under eye pressure during blinking to allow for the delivery of one or more agents to the eye, dynamic cosmetic eye enhancement and/or dynamic rotational misalignment correction. The one or more agents may include therapeutic agents, pharmacological agents. Delivery of the agent may be via a single one-way check valve.

### SUMMARY OF THE INVENTION

The present invention is set out in the appended claims. In many embodiments, a fluidic module may be embedded in a soft contact lens that may be filled with a concentrated solution of a drug to be delivered into the eye in a sustained manner.

In many embodiments, the module comprises a hydrophobic material having channels formed therein, such that a surface tension of the aqueous solution within the channels inhibits release of therapeutic agent, such as a drug, through the one or more channels. The surface tension of the aqueous solution within the channel can inhibit diffusion of the therapeutic agent through the channel, which can be in addition to decreased diffusion through the channel related to the cross-sectional area of the channel. The channels may comprise a cross-sectional area and optionally a length, such that therapeutic agent is released through the channels when pressure of the eyelid increases, and release is inhibited when pressure of the eyelid decreases. In many embodiments, the contact lens is configured to inhibit release of the therapeutic agent when the contact lens comprises a free floating configuration, for example when stored in a contact lens solution, such that the storage time of the contact lens can be increased substantially over contact lenses relying on a diffusion based release of therapeutic agent. The therapeutic agent may comprise one or more of many substances capable of providing a therapeutic benefit to the eye, such as substances capable of treating one or more of dry eye, uveitis. The therapeutic agent may comprise one or more of many therapeutic agents, such as a drug, a surfactant, a solution, a lipid or a component of an artificial tear, for example.

In many embodiments, the openings are sized to provide a gated release of therapeutic agent in response to pressure of the eyelid, in which surface tension extending across each of the plurality of openings inhibits diffusion of therapeutic agent through the opening, and a pressure of the eyelid urges fluid of the container through the plurality of openings. The gated release has the advantage of inhibiting diffusion through a cross-sectional area of the opening, such that storage life of the contact lens in solution can be extended substantially. The gated release also has the advantage of providing therapeutic agent, such that the wearer receives an amount of therapeutic agent in response to blinking in order for the user to control the amount of therapeutic agent provided. Such embodiments can be particularly well suited for the delivery of therapeutic agents to treat dry eye, for example.

In a first aspect, embodiments comprise a soft contact lens. The soft contact lens comprises a container comprising a plurality of openings sized to release a therapeutic agent, and a soft contact lens material encapsulating the module.

In many embodiments, the soft contact lens further comprises a module. The module comprises the container. The module may comprise a plurality of anchors to anchor the module in the soft contact lens material. The module comprises a barrier material to inhibit release of the therapeutic agent.

In many embodiments, the material of the module comprises an optically transparent material extending across at least a portion of an optically corrective portion of the contact lens, and one or more of the plurality of anchors extends at least partially within the optically used portion of the lens.

In many embodiments, the material comprises an index of refraction similar to the soft contact lens material in order to inhibit light scatter.

In many embodiments, the openings are sized to provide a gated release of therapeutic agent in response to pressure of the eyelid, in which surface tension extending across each of the plurality of openings inhibits diffusion of therapeutic agent through the opening, and a pressure of the eyelid urges fluid of the container through the plurality of openings.

In many embodiments, the container comprises a hydrophobic material, and the openings are sized to release the therapeutic agent in response to pressure of the eyelid and to inhibit release of the therapeutic agent when the contact lens comprises a free floating configuration.

In many embodiments, the openings are sized to inhibit diffusion of the therapeutic agent through the opening in response to a surface tension of a solution comprising the therapeutic agent.

In many embodiments, the openings of the container comprise a length extending along a thickness of the container wall and are dimensioned with a cross sectional area in to release therapeutic agent in response to pressure of the eyelid and inhibit diffusion of the therapeutic agent through the cross-sectional area.

In many embodiments, a maximum dimension across the cross-sectional area comprises no more than about 50 nm, for example no more than about 5 nm.

In many embodiments, the length is sized to allow a therapeutic amount of fluid comprising the therapeutic agent to be forced through the opening with pressure of the eyelid.

In many embodiments, the free floating configuration comprises a configuration of the contact lens placed in a solution of a storage container.

In many embodiments, this fluidic module is comprised of flexible membranes and is matched in refractive index to the lens substrate so that it does not cause any optical disturbance or changes in refractive property of the contact lens.

In many embodiments, the one or more membranes comprising the wall of the fluidic module is drilled with one or more of a precision drill, a laser, an electron beam, a water jet, or an etching process, in order to form submicron size holes, such as nanometer sized holes.

In many embodiments, the diameter of the holes is such that the rate of drainage through these holes is negligible under normal conditions of atmospheric pressure and body temperature.

In many embodiments, the diameter of the holes is sized, for example adjusted, so that the rate of drainage increases when eyelids put pressure on the contact lens in the eye during blinking, for example.

In many embodiments, the drainage of the drug solution/suspension occurs in pulses during daytime, immediately following blinking, and continuously during down-gaze, at a lower rate.

In many embodiments, the contact lens bearing the drug eluting module is designed to be removed before going to sleep.

In many embodiments, the soft contact lens has a diameter of 10-14 mm and the embedded fluidic module has a diameter of 3-12 mm.

In many embodiments, the fluidic module is barrel shaped, with a height of 10-200 microns, preferably 50-150 microns. Alternatively, the fluidic module may comprise an annular shape, or a plurality of reservoirs located away from an inner optical region of the contact lens.

In many embodiments, the fluidic module is comprised of membranes that are impermeable to water and other hydrophilic liquids.

In many embodiments, the wall thickness of the membranes comprising the fluidic module varies from 5-25 microns.

In many embodiments, the posterior (cornea facing) wall of the fluidic module is penetrated with a number of submicron sized holes, of diameter in the range 100-500 nm, designed to allow minimal drainage under normal handling and storage conditions, but allow enhanced drainage when the module is pressurized. In many embodiments, the diameter of the holes is within a range from about 0.5 nm to about 5 nm, in order to provide decreased drainage and inhibit diffusion of the therapeutic agent.

In many embodiments, the number of the submicron holes is in the range of 10² (100) to 10⁶ (1,000,000) per module.

In many embodiments, the volume of the fluidic module is in the range 1-10 microliter, preferably, 3-8 microliter.

In many embodiments, the holes are only placed on the wall of the module in contact with the cornea in order to deliver the drug into the post tear film, that persists for up to 20-30 minutes before being drained into the sub-conjunctival nasolacrimal glands.

In many embodiments, this module is filled with a solution of an ocular drug at a concentration in the range 1-100 g/L (grams per liter), or 1-300 x 10⁻³ M/L (moles per liter).

In many embodiments, the loading of drugs in a single module prior to encapsulation into the contact lens is in the range 50-500 micrograms.

In many embodiments, it is estimated that approximately 6 micrograms of Timolol (timolol maleate) is required to be delivered on the cornea every day for treatment of glaucoma caused by enhancement of intraocular pressure.

In many embodiments, a drug eluting lens comprising a fluidic module may be used for up to 1 month or longer for sustained delivery of this drug.

In many embodiments, it is also expected that 80% or more of the drug eluted from the contact lens is actually delivered on the cornea, because of the specific placement of the drainage holes.

In many embodiments, the force exerted by eyelids on the contact lens during blinking is in the range of 4-50 millinewtons, preferably, 8-30 millinewtons.

The diameter of the drain holes on the wall of the fluidic module is adjusted so that proper drainage rate is achieved when the module is placed under this pressure.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 shows a top view of the fluidic module, comprising an optically centered chamber, in accordance with embodiments;
Figure 2 shows a side view of the contact lens as in Figure 1, comprising the fluidic module embedded in a soft contact lens, in accordance with embodiments; and
Figure 3 shows a contact lens comprising a drug delivery module having an annular reservoir container, in accordance with embodiments;

### DETAILED DESCRIPTION

The embodiments disclosed herein are well suited for combination with many prior contact lenses and therapeutic agents.

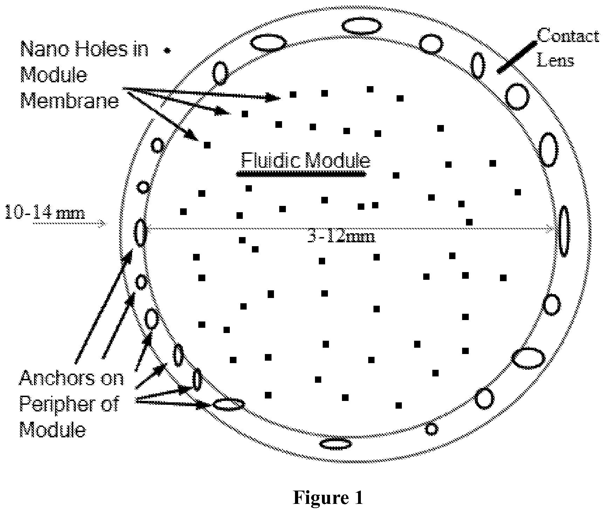

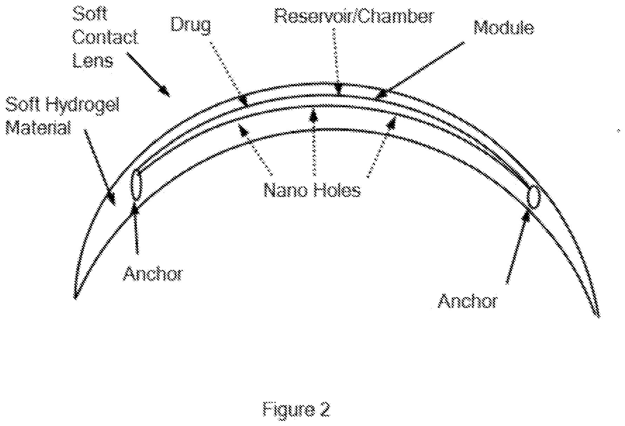

Figure 1 shows a top view of the fluidic module, comprising an optically centered chamber, and Figure 2 shows a side view of the contact lens as in Figure 1, comprising the fluidic module embedded in a soft contact lens, in accordance with embodiments.

In many embodiments, a soft hydrogel contact lens comprises a reservoir module embedded inside the contact lens to release a therapeutic agent such as drug. The therapeutic agent may comprise lipid to inhibit evaporation of the tear film, in order to treat dry eye, for example. The soft hydrogel material may comprise a silicone hydrogel material that readily releases the therapeutic agent to the eye, for example to the tear film, when release from the module. The reservoir module comprises a wall to contain and inhibit release of the drug, and holes sized to release the drug. The holes can be sized large, e.g. greater than 500 nm to release the drug with diffusion or when the eyelid blinks and applies pressure to the module. Alternatively, the holes can be sized smaller than 500 nm, for example smaller than 50 nm, for further example smaller than 5 nm, such that drug is only released when the user blinks and in response to the blinking pressure, depending on the molecular size of the therapeutic agent. In many embodiments, the module comprises a hydrophobic barrier material, such that the surface tension of water within the small holes inhibits diffusion of the drug molecules through the openings. The barrier material may comprise one or more of many materials capable of inhibiting release of the therapeutic agent through the material itself. Once released through the holes, the drug diffuses through the hydrogel material to the eye. The module comprises a transparent material, and may comprise a plurality of outer anchors to hold the module in the soft hydrogel material. The anchors comprise loops of materials or openings in a flange of the module sized to receive hydrogel material of the contact lens and anchor the module when embedded within the lens. The contact lens has a diameter from about 10-14 mm and the module has a diameter within a range from about 3-12 mm.

In many embodiments, the module comprises a sufficient number of openings to release a therapeutic amount of therapeutic agent over the time the lens is worn, in order to provide a therapeutic benefit. The volume of the reservoir, and the size and number of openings can be configured to release the therapeutic amount in response to blinking of the eye. The reservoir can be sized to provide the therapeutic amount over a predetermined time, for a number of blinks each day, for example.

In many embodiments, each of the plurality of openings comprises a subnanometer hole in a hydrophobic membrane film, such that one or more of a permeation rate or a diffusion is substantially lower than a corresponding permeation rate of a similar hole in a similar hole in a hydrophilic membrane film. In many embodiments, the permeation of diffusion rate is at least about one order of magnitude lower, such as at least about two orders of magnitude lower and can be within a range from about 1-4 orders of magnitude lower, for example 3 to 4 orders of magnitude lower.

In many embodiments, each of the plurality of openings is sized to a therapeutic agent to be delivered through said each opening.

For example, the hole size to stop diffusion of pure water is about 0.5, which can be referred to as a critical size of the hole for pure water. In many embodiments, the critical size of the gate is larger for therapeutic agents having a bigger molecular size. A larger therapeutic agent can have a larger diameter hole than pure water in order to deliver therapeutic amounts as described herein.

The membrane of the module comprises a biocompatible compatible material, and has an index preferably substantially the same as the fluid and the contact lens itself, in the range 1.44 - 1.55, or within the range from 1.40 to 1.55, for example.

The membrane may be of the same thickness throughout, or it may have a thickness profile, contoured to control its rigidity or flexibility along the dimensions of the membrane.

The membrane is preferably a fluorocarbon, a polyester, a polyurethane, a polyether, a polyimide, a polyamide, an acrylate or methacrylate ester, or a copolymer bearing these functionalities.

The module may comprise on or more of many optically transmissive materials, such as one or more of a plastic, a polymer, a thermo plastic, a fluoropolymer a nonreactive thermoplastic fluoropolymer, or polyvinylidene difluoride (hereinafter "PVDF"), for example. In many embodiments, the material comprises an optically transmissive hydrophobic material, for example.

The walls of the module may either be composed of the same material as the membrane on the two sides, or it may be made of a different material.

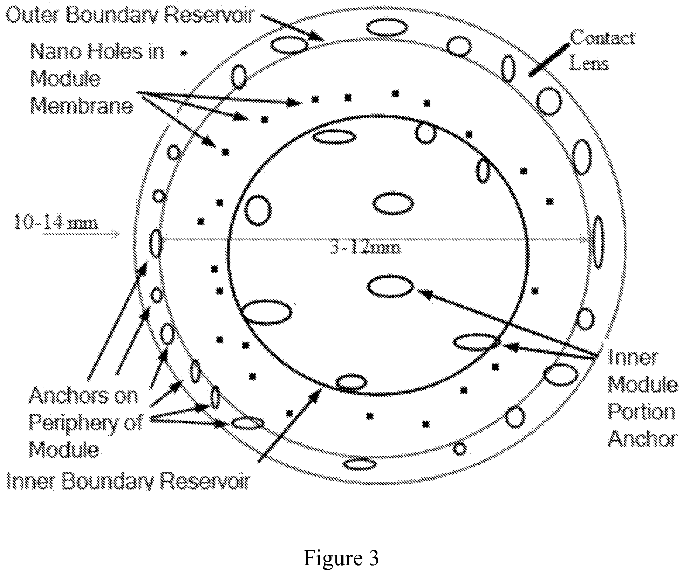

Figure 3 shows a contact lens comprising a drug delivery module having an annular reservoir container. In many embodiments, the module comprises an annular shape with the reservoir located away from an optical axis of the eye and entrance pupil of the eye, in order to inhibit refractive changes of the contact lens when fluid is released from the module. Alternatively, the module may comprise a plurality of reservoirs located away from a central optical axis of the lens that approximately corresponds to an optical axis of the eye. The outer reservoir chamber located away from the optical axis may comprise an annular ring shaped chamber, or a plurality of chambers arranged with a substantially annular profile, for example. Each of the plurality of reservoir chambers may comprise a plurality of holes as described herein, for example.

In many embodiments, the one or more reservoir containers located away from the optical axis comprises an inner boundary wall located toward the optical axis and an outer boundary wall located away from the optical axis. A first plurality of inwardly located anchors can be located inward from the inner boundary wall, and a second plurality of outwardly located anchors can be located outward from the outer boundary wall. Each of the anchors may comprise an opening formed in a layer of material such that the soft contact lens material extends through the opening. The anchors may comprise loops, apertures or other structures such as branches or struts of configured to contact the soft contact lens material and anchor the module to the soft contact lens material. The anchors and modules may comprise optically transparent materials having an index of refraction that substantially corresponds to the index of refraction of the soft contact lens hydrogel material, in order to inhibit light scatter.

In many embodiments, and inner portion of the module extends between the inner module wall extending circumferentially around the inner portion.

The module may comprise one or more of many materials, for example.

A soft contact lens comprised of a hydrogel that may be a cross-linked polyhydroxy ethyl methacrylate network or a silicone-hydrogel copolymer is embedded with a sealed fluidic module comprised of impermeable walls penetrated with a plurality of through holes, each of diameter in the range of 100 nm to 500 nm.

Preferably, the holes are drilled exclusively on the surface of the fluidic module that faces the corneal surface.

These holes may be drilled by reactive ion etching or by etching using a solution, through a mask to control the hole size.

The fluidic module is filled with a solution of a desired ophthalmic drug that is required to be administered on the surface of the cornea, so that it may be transported across the cornea into the aqueous humor of the eye.

The solution has a viscosity in the range of 10-100 cps, preferably in the range 20-80 cps.

Preferably, the drug solution is miscible with the tear fluid, so that the mixture remains clear.

The viscosity of the solution is adjusted so that transport of this solution through the holes is minimized by external air pressure when the lens comprising the embedded fluidic module is stored under normal conditions of atmospheric pressure and temperature.

The concentration of the drug in the solution is in the range of 1-300 millimoles/L.

Preferably, the concentration of the drug in the solution is in the range 50-100 millimoles/L

The fluidic module is embedded in the soft contact lens such that the module is close to the bottom of the contact lens.

Preferably there is a thin layer of contact lens material below the fluidic module, its thickness being in the range of 5-10 microns.

Pressurization of the fluidic module due to blink applied pressure by the eyelids causes forced ejection of fluid from the fluidic module that then diffuses through the contact lens material and enters the post tear film and stays in contact with the cornea for a period of up to 20-30 minutes.

An incremental volume of drug laden solution is thus delivered on the cornea by every blink.

As the pressure on the contact lens is relieved after the blink, the fluidic module comes under negative pressure, since some of the fluid has been ejected from the module during the blink period.

The negative pressure induces tear fluid to enter into the fluidic module and equalize the pressure difference created by ejection of the drug solution

The tear fluid causes the drug solution to become diluted.

This process reduces the incremental amount of drug delivered per blink as more drug is delivered.

The trans-corneal transport of the drug competes with the delivery of the drug via blinks, so that an equilibrium drug concentration is established in the post tear film in contact with the cornea, underneath the contact lens

The diameter of the fluidic module is in the range 3-12 mm, preferably 8-10 mm.

The thickness of the fluidic module is 10-200 microns, preferably 50-150 microns

The thickness of the membrane comprising the wall of the membrane is in the range of 5-25 microns.

The volume of the fluidic module is 3-12 microliters, preferably 5-8 microliters.

The drug loading per module is therefore in the range of 75-240 micrograms

The amount of drug solution ejected out of the fluidic module per blink is approximately 5-10 pL, depending on the viscosity of the solution and the diameter of the holes.

The amount of drug delivered per blink is 75-300 picograms.

Approximately 0.025 to 0.1 micrograms will be delivered before the post tear film is cleared by the eye.

Since the average blink rate is about 10 blinks per minute, there are approximately 1.2X104 blinks per 20 hours, delivering about 0.06-0.12 microliters of fluid per day.

This means that the drug reservoir will be diluted by as much as 15% or as little as 5% per day, depending on the number of hours of wear of the contact lens and its design, as well as the blink rate of the individual.

The drug concentration will therefore be reduced to 85-95% per day of use.

This rate of dilution translates to an effective use period of 2 - 7 days, assuming that a dosage variation of 25-30% is acceptable.

This variation will produce a corresponding variation in bioavailability of the drug in the anterior chamber

Such a variation is substantially less than that achieved by twice daily topical applications

Thus a broad range of delivery dosages and use periods will be achieved by this device.

### EXAMPLES OF THERAPEUTIC AGENTS AND STUDIES SUITABLE FOR INCORPORATION IN ACCORDANCE WITH EMBODIMENTS DISCLOSED HEREIN

A person of ordinary skill in the art can modify prior therapeutic agents and delivery devices in accordance with the teachings disclosed herein.

### Clinical Applications

Among clinical applications are as follows.

### Bacterial of fungal keratitis

Sight-threatening conditions, such as microbial keratitis, require rapid and sustained delivery of high levels of medication to the affected tissues. Currently, patients with severe microbial keratitis are hospitalized to ensure continuous delivery of therapeutic levels of antibiotic through round-the-clock dosing. Management of these patients could be dramatically improved through the use of an effective drug delivery system, ensuring continuously high antibiotic levels. Contact lens delivery of antibiotics has been investigated, showing some improvement over delivery via eyedrops. Fungal keratitis is a major cause of blindness in tropical developing countries, and requires sustained delivery of antifungal agents for their management and cure

### Glaucoma

A feasibility study investigating efficacy and toxicity of contact lenses that were passively impregnated with timolol maleate and brimonidine tartrate found IOP reductions equivalent to conventional therapy and no toxicity.

### Dry eye

Myobium gland dysfunction has been associated with a deficiency of phospholipids in the tear film. Drug-eluting contact lenses have been used to provide controlled release of phospholipids. Phospholipid-eluting contact lenses may provide an effective treatment for some forms of dry eye, and possibly can improve end-of-day dryness in contact lens wearers by stabilizing the tear film and enhancing lens wettability. This may be especially helpful in wearers of silicone hydrogel lenses, which sequester lipids due to the hydrophobic nature of their surfaces

### Allergy or uveitis

The eye is especially vulnerable to auto-immune disorders such as uveitis, requiring sustained administration of immuno-modulators or immuno-suppresants for their control. These are required to be administered topically or via sustained drug delivery, because of their systemic toxicity. The use of ketotifen-containing contact lenses for the management of ocular allergy has been investigated experimentally, and has also undergone several clinical trials addressing safety and efficacy.

Based on the teachings disclosed herein, a person of ordinary skill in the art can determine dimensions of the openings, the thickness of the container wall, the number of openings, and the reservoir volume in order to provide therapeutic amounts of the therapeutic agent over a predetermined amount of time. For example, a pressure of the eyelid with a blink can be determined, and amounts of therapeutic agent released through each opening with each blink determined. The number of times a person blinks during a day can be used to determine the amount of therapeutic agent released per day.

The amount of therapeutic agent released through the plurality of openings during non-blink times can be determined based on Fick's law of diffusion for channels appropriately sized to allow diffusion, for example having a diameter across of at least about 50 nm. Alternatively or in combination, the amount of therapeutic agent released through the plurality of openings during non-blink times can be determined for channels appropriately sized to inhibit diffusion through the channels and to release therapeutic agent in response to eyelid pressure, for example channels having a diameter across of no more than about 50 nm, for example, depending on the molecular size of the therapeutic agent. The gated release of therapeutic agent in response to each eye blink can be studied in human subjects with known study designs suitable for incorporation in accordance with embodiments disclosed herein.

In many embodiments, a therapeutic agent can be identified, and the molecular size of the therapeutic agent determined based on published data. Based on this published size, a plurality of holes as described herein can be formed in a barrier material, in which each of the holes comprises a diameter corresponding to the therapeutic agent, for example slightly larger than a molecular diameter of the therapeutic agent, and studies conducted with known materials and apparatus to determine the amount of therapeutic agent released.

An example of molecular gating with hydrophobic surfaces suitable for incorporation in accordance with embodiments described herein is described in Principles of Gating Mechanisms of Ion Channels, by Oliver Beckstein, a thesis submitted in partial fulfillment of the requirements for the degree of Doctor of Philosophy at the University of Oxford, Michelmas 2004, available on the world wide web at (sbcb.bioch.ox.ac.uk/users/oliver/download/Thesis/OB_thesis_2sided.pdf).

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention.

## Claims

1. A soft contact lens comprising:
a container comprising a plurality of openings sized to release a therapeutic agent; and
a soft contact lens material encapsulating the container wherein the openings are sized to provide a gated release of therapeutic agent in response to pressure of the eyelid, in which surface tension extending across each of the plurality of openings inhibits diffusion of therapeutic agent and wherein a pressure of the eyelid urges fluid of the container through the plurality of openings.

2. A soft contact lens as in claim 1, further comprising a module, the module comprising the container, wherein the module comprises a plurality of anchors to anchor the module in the soft contact lens material and wherein the module comprises a barrier material to inhibit release of the therapeutic agent.

3. A soft contact lens as in claim 2, wherein a material of the module comprises an optically transparent material extending across at least a portion of an optically corrective portion of the contact lens and wherein one or more of the plurality of anchors extends at least partially within the optically used portion of the lens and wherein the material comprises an index of refraction similar to the soft contact lens material in order to inhibit light scatter.

4. A soft contact lens as in claim 1, wherein the container comprises a hydrophobic material, and wherein the openings are sized to release the therapeutic agent in response to pressure of the eyelid and to inhibit release of the therapeutic agent when the contact lens comprises a free floating configuration, wherein the openings of the container comprise a length extending along a thickness of the container wall and are dimensioned with a cross sectional area in to release therapeutic agent in response to pressure of the eyelid and inhibit diffusion of the therapeutic agent through the cross-sectional area optionally wherein a maximum dimension across the cross-sectional area comprises no more than about 50 nm, optionally wherein the length is sized to allow a therapeutic amount of fluid comprising the therapeutic agent to be forced through the opening with pressure of the eyelid.

5. A soft contact lens as in claim 4, wherein the free floating configuration comprises a configuration of the contact lens placed in a solution of a storage container.

6. A soft contact lens as in claim 1, further comprising a fluid filled module filled with a solution of said drug at a concentration of 1-300 millimoles per liter.

7. The soft contact lens of claim 6 wherein:
said module is comprised of membranes wherein said membranes are penetrated by holes of diameter in the range of 100-500 nanometers; or
said module is of diameter in the range of 5 mm to 10 mm; or
said solution cannot permeate through said membrane of claim 2 except through said holes.

8. The soft contact lens of claim 7 wherein said holes are added exclusively on the membrane facing the cornea of the eye fitted with said lens of claim 1.

9. The soft contact lens of claim 6 wherein at least one of:
said lens elutes drugs substantially only when said lens is under a compressive force greater than 8 millinewtons;
said solution has a preferred range of concentration of 50 -100 millimoles per liter;
said module comprises membranes who thickness is in the range of 10-30 microns;
said drug is in the range of 50-500 micrograms, preferably 50-250 micrograms;
said solution is of volume 3-12 microliters;
said module ejects a volume of solution of a drug equal to 5-10 picoliters per blink when said contact lens of claim 1 is fitted in the eye of a wearer;
the module comes under an eyelid pressure of 3-20 mm of mercury during a blink;
said module comes under negative pressure during the inter-blink period;
tear fluid enters said module through the holes on said membrane of claim 8 during the inter-blink period;
said module does not substantially affect the refractive properties of said lens.

10. A soft contact lens as in claim 1, further comprising a fluidic module and wherein said lens may be worn continuously for a period not exceeding 11 months.

11. A soft contact lens as in claim 1, further comprising a fluidic module and wherein said lens is replaced at least once a year.

12. The soft contact lens of any one of the preceding claim wherein said lens is designed for correction of myopia, hyperopia, astigmatism, prismatic errors and any combinations thereof.

13. A method of forming a soft contact lens comprising:
providing a container comprising a plurality of openings sized to release a therapeutic agent; and
encapsulating the container in a soft contact lens material the container wherein the openings are sized to provide a gated release of therapeutic agent in response to pressure of the eyelid, in which surface tension extending across each of the plurality of openings inhibits diffusion of therapeutic agent and wherein a pressure of the eyelid urges fluid of the container through the plurality of openings.

## Patentansprüche

1. Weiche Kontaktlinse, die Folgendes umfasst:
einen Container mit mehreren Öffnungen, die zum Freisetzen eines therapeutischen Mittels bemessen sind; und
ein weiches Kontaktlinsenmaterial, das den Container verkapselt, wobei die Öffnungen so bemessen sind, dass sie eine torgesteuerte Freisetzung von therapeutischem Mittel als Reaktion auf Druck des Augenlids bereitstellen, wobei Oberflächenspannung über alle der mehreren Öffnungen Diffusion von therapeutischem Mittel hemmt und wobei ein Druck des Augenlids Flüssigkeit des Containers durch die mehreren Öffnungen drängt.

2. Weiche Kontaktlinse nach Anspruch 1, die ferner ein Modul umfasst, wobei das Modul den Container umfasst, wobei das Modul mehrere Anker zum Verankern des Moduls in dem weichen Kontaktlinsenmaterial umfasst und wobei das Modul ein Sperrmaterial zum Hemmen der Freisetzung des therapeutischen Mittels umfasst.

3. Weiche Kontaktlinse nach Anspruch 2, wobei ein Material des Moduls ein optisch transparentes Material umfasst, das sich über wenigstens einen Teil eines optisch korrigierenden Teils der Kontaktlinse verläuft und wobei sich ein oder mehrere aus der Mehrzahl von Ankern wenigstens teilweise in den optisch genutzten Teil der Linse erstrecken und wobei das Material einen Brechungsindex ähnlich dem des weichen Kontaktlinsenmaterials hat, um Lichtstreuung zu hemmen.

4. Weiche Kontaktlinse nach Anspruch 1, wobei der Container ein hydrophobes Material umfasst und wobei die Öffnungen so bemessen sind, dass sie das therapeutische Mittel als Reaktion auf den Druck des Augenlids freisetzen und die Freisetzung des therapeutischen Mittels hemmen, wenn die Kontaktlinse eine freischwebende Konfiguration hat, wobei die Öffnungen des Containers eine Länge haben, die sich über eine Dicke der Containerwand erstreckt, und mit einer solchen Querschnittsfläche dimensioniert sind, dass therapeutisches Mittel als Reaktion auf Druck des Augenlids freigesetzt und Diffusion des therapeutischen Mittels durch die Querschnittsfläche gehemmt wird, wobei optional ein maximales Maß über die Querschnittsfläche maximal etwa 50 nm ist, wobei die Länge optional so bemessen ist, dass eine therapeutische Menge Flüssigkeit, die das therapeutische Mittel umfasst, durch die Öffnung mit Druck des Augenlids gezwungen werden kann.

5. Weiche Kontaktlinse nach Anspruch 4, wobei die freischwebende Konfiguration eine Konfiguration der Kontaktlinse in einer Lösung eines Speichercontainers platziert umfasst.

6. Weiche Kontaktlinse nach Anspruch 1, die ferner ein flüssigkeitsgefülltes Modul umfasst, gefüllt mit einer Lösung des genannten Arzneimittels mit einer Konzentration von 1-300 Millimol pro Liter.

7. Weiche Kontaktlinse nach Anspruch 6, wobei:
das genannte Modul aus Membranen besteht, wobei die genannten Membranen mit Löchern mit einem Durchmesser im Bereich von 100-500 Nanometern perforiert sind; oder
das genannte Modul einen Durchmesser im Bereich von 5 mm bis 10 mm hat; oder
die genannte Lösung nicht durch die genannte Membran nach Anspruch 2 außer durch die genannten Löcher permeieren kann.

8. Weiche Kontaktlinse nach Anspruch 7, wobei die genannten Löcher ausschließlich auf der Membran hinzugefügt werden, die der Hornhaut des mit der genannten Linse von Anspruch 1 ausgestatteten Auges zugewandt ist.

9. Weiche Kontaktlinse nach Anspruch 6, wobei wenigstens eines der Folgenden gilt:
die genannte Linse eluiert Artneimittel im Wesentlichen nur dann, wenn die genannte Linse unter einer Druckkraft von mehr als 8 Millinewton ist;
die genannte Lösung hat einen bevorzugten Konzentrationsbereich von 50-100 Millimol pro Liter;
das genannte Modul umfasst Membranen, deren Dicke im Bereich von 10-30 Mikron liegt;
das genannte Arzneimittel ist im Bereich von 50-500 Mikrogramm, vorzugsweise von 50-250 Mikrogramm;
die genannte Lösung hat ein Volumen von 3-12 Mikrolitern;
das genannte Modul ejiziert ein Lösungsvolumen eines Arzneimittels von 5-10 Pikolitern pro Blinken, wenn die genannte Kontaktlinse nach Anspruch 1 im Auge eines Trägers sitzt;
das Modul kommt bei einem Blinken unter einen Augenliddruck von 3-20 mm Quecksilbersäule;
das genannte Modul kommt während der Zwischenblinkperiode unter Negativdruck;
Tränenflüssigkeit tritt in das genannte Modul durch die Löcher auf der genannten Membran nach Anspruch 8 während der Zwischenblinkperiode ein;
das genannte Modul beeinflusst die Brechungseigenschaften der genannten Linse nicht wesentlich.

10. Weiche Kontaktlinse nach Anspruch 1, die ferner ein fluidisches Modul umfasst, wobei die genannte Linse für eine Periode von maximal 11 Monaten ständig getragen werden kann.

11. Weiche Kontaktlinse nach Anspruch 1, die ferner ein fluidisches Modul umfasst, wobei die genannte Linse wenigstens einmal im Jahr ausgetauscht wird.

12. Weiche Kontaktlinse nach einem der vorherigen Ansprüche, wobei die genannte Linse zum Korrigieren von Myopie, Hyperopie, Astigmatismus, prismatischen Fehlern und beliebigen Kombinationen davon ausgelegt ist.

13. Verfahren zum Bilden einer weichen Kontaktlinse, das Folgendes beinhaltet:
Bereitstellen eines Containers, der mehrere Öffnungen aufweist, die zum Freisetzen eines therapeutischen Mittels bemessen sind; und
Verkapseln des Containers in einem weichen Kontaktlinsenmaterial, wobei die Öffnungen so bemessen sind, dass sie eine torgesteuerte Freisetzung von therapeutischem Mittel als Reaktion auf Druck des Augenlids bereitstellen, wobei Oberflächenspannung über alle der mehreren Öffnungen Diffusion von therapeutischem Mittel hemmt und wobei ein Druck des Augenlids Flüssigkeit des Containers durch die mehreren Öffnungen drängt.

## Revendications

1. Lentille de contact souple comprenant :
un contenant comprenant une pluralité d'ouvertures dimensionnées pour libérer un agent thérapeutique ; et
un matériau de lentille de contact souple encapsulant le contenant, les ouvertures étant dimensionnées pour assurer une libération contrôlée d'agent thérapeutique en réponse à une pression de la paupière, la tension superficielle s'étendant à travers chacune de la pluralité d'ouvertures inhibant la diffusion d'agent thérapeutique et une pression de la paupière faisant passer le fluide présent dans le contenant par la pluralité d'ouvertures.

2. Lentille de contact souple selon la revendication 1, comprenant en outre un module, le module comprenant le contenant, dans laquelle le module comprend une pluralité d'ancres pour ancrer le module dans le matériau de lentille de contact souple et dans laquelle le module comprend un matériau de barrière pour inhiber la libération de l'agent thérapeutique.

3. Lentille de contact souple selon la revendication 2, dans laquelle un matériau du module comprend un matériau optiquement transparent s'étendant à travers au moins une partie d'une partie optiquement correctrice de la lentille de contact et dans laquelle une ou plusieurs de la pluralité d'ancres s'étend au moins partiellement à l'intérieur de la partie utilisée optiquement de la lentille et dans laquelle le matériau a un indice de réfraction similaire à celui du matériau de la lentille de contact souple afin d'inhiber la diffusion de la lumière.

4. Lentille de contact souple selon la revendication 1, dans laquelle le contenant comprend un matériau hydrophobe, et dans laquelle les ouvertures sont dimensionnées pour libérer l'agent thérapeutique en réponse à une pression de la paupière et pour inhiber la libération de l'agent thérapeutique lorsque la lentille de contact est disposée dans une configuration de flottaison libre, dans laquelle les ouvertures du contenant ont une longueur s'étendant le long d'une épaisseur de la paroi du contenant et sont dimensionnées avec une section transversale de façon à libérer l'agent thérapeutique en réponse à une pression de la paupière et inhiber la diffusion de l'agent thérapeutique à travers la section transversale, optionnellement dans laquelle une dimension maximum à travers la section transversale ne dépasse pas environ 50 nm, optionnellement dans laquelle la longueur est dimensionnée pour permettre le passage forcé d'une quantité thérapeutique de fluide comprenant l'agent thérapeutique par l'ouverture sous l'effet d'une pression de la paupière.

5. Lentille de contact souple selon la revendication 4, dans laquelle la configuration de flottaison libre comprend une configuration dans laquelle la lentille de contact est placée dans une solution présente dans un contenant de conservation.

6. Lentille de contact souple selon la revendication 1, comprenant en outre un module rempli de fluide rempli d'une solution dudit médicament à une concentration de 1 à 300 millimoles par litre.

7. Lentille de contact souple selon la revendication 6, dans laquelle :
ledit module est constitué de membranes, lesdites membranes étant pénétrées par des trous ayant un diamètre de 100 à 500 nanomètres ; ou
ledit module a un diamètre de 5 mm à 10 mm ; ou
ladite solution ne peut traverser par perméation ladite membrane selon la revendication 2 que par lesdits trous.

8. Lentille de contact souple selon la revendication 7, dans laquelle lesdits trous sont ajoutés exclusivement sur la membrane faisant face à la cornée de l'oeil portant ladite lentille selon la revendication 1.

9. Lentille de contact souple selon la revendication 6, dans laquelle au moins l'une des déclarations suivantes s'applique :
ladite lentille élue des médicaments quasi-uniquement lorsque ladite lentille est sous une force de compression supérieure à 8 millinewtons ;
ladite solution a une plage de concentrations préférée de 50 à 100 millimoles par litre ;
ledit module comprend des membranes dont l'épaisseur est de 10 à 30 microns ;
ledit médicament est présent dans une quantité de 50 à 500 microgrammes, préférablement de 50 à 250 microgrammes ;
ladite solution est présente dans un volume de 3 à 12 microlitres ;
ledit module éjecte un volume de solution d'un médicament égal à 5 à 10 picolitres par clignement lorsque ladite lentille de contact selon la revendication 1 est présente sur l'oeil d'un porteur ;
le module est soumis à une pression de paupière de 3 à 20 mm de mercure pendant un clignement ;
ledit module est soumis à une pression négative pendant la période entre les clignements ;
le fluide lacrymal pénètre dans ledit module par les trous présents sur ladite membrane selon la revendication 8 pendant la période entre les clignements ;
ledit module n'influence pas sensiblement les propriétés de réfraction de ladite lentille.

10. Lentille de contact souple selon la revendication 1, comprenant en outre un module fluidique, et ladite lentille pouvant être portée sans interruption pendant une période ne dépassant pas 11 mois.

11. Lentille de contact souple selon la revendication 1, comprenant en outre un module fluidique, et ladite lentille étant remplacée au moins une fois par an.

12. Lentille de contact souple selon l'une quelconque des revendications précédentes, ladite lentille étant conçue pour corriger la myopie, l'hypermétropie, l'astigmatisme, les erreurs prismatiques et n'importe quelles combinaisons de ceux-ci.

13. Procédé de formation d'une lentille de contact souple, comprenant :
la fourniture d'un contenant comprenant une pluralité d'ouvertures dimensionnées pour libérer un agent thérapeutique ; et
l'encapsulation du contenant dans un matériau de lentille de contact souple, les ouvertures étant dimensionnées pour assurer une libération contrôlée d'agent thérapeutique en réponse à une pression de la paupière, la tension superficielle s'étendant à travers chacune de la pluralité d'ouvertures inhibant la diffusion d'agent thérapeutique et une pression de la paupière faisant passer le fluide présent dans le contenant par la pluralité d'ouvertures.
